(19) Europäisches Patentamt
European Patent Office
Office européen des brevets

(11) **EP 3 656 440 A1**

(12) **EUROPEAN PATENT APPLICATION**

(43) Date of publication:
**27.05.2020 Bulletin 2020/22**

(21) Application number: **18207973.1**

(22) Date of filing: **23.11.2018**

(51) Int Cl.:
*A61N 1/40* (2006.01)    *A61N 7/02* (2006.01)
*A61N 1/04* (2006.01)    *A61N 1/06* (2006.01)
*A61B 90/50* (2016.01)   *A61N 7/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **GLEICH, Bernhard
  5656 AE Eindhoven (NL)**
• **BOERNERT, Peter
  5656 AE Eindhoven (NL)**

• **EWALD, Arne
  5656 AE Eindhoven (NL)**
• **FLAESCHNER, Nick
  5656 AE Eindhoven (NL)**
• **SCHMALE, Ingo
  5656 AE Eindhoven (NL)**
• **OVERWEG, Johannes Adrianus
  5656 AE Eindhoven (NL)**
• **GRAESSLIN, Ingmar
  5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **HEADSET FOR GENERATING AN RF EM FIELD AND FOCUSED ULTRASOUND WAVES**

(57)    The invention provides for a medical apparatus (100) comprising a headset (102) with one or more coils (112) for generating a radio frequency electromagnetic field and one or more ultrasound transmitters (110) for generating focused ultrasound waves. A processor (128) controls the one or more coils to generate the radio frequency electromagnetic field and the one or more ultrasound transmitters to generate the focused ultrasound waves, wherein a ratio of electric power density of the electromagnetic field to acoustic power density of the ultrasound waves is limited to lie within a range from 0.5 to 2.

FIG. 1

EP 3 656 440 A1

**Description**

FILED OF THE INVENTION

The invention relates to a medical apparatus, a computer program product and a method for generating a radio frequency electromagnetic field and focused ultrasound waves.

BACKGROUND OF THE INVENTION

Electrical stimulation of the brain is a promising new curative approach for various medical conditions, e.g. for stroke rehabilitation or serious depression disorder. However, one of the main problems of electrical brain stimulation is that no currently available apparatus allows targeting of small localized brain volumes without an invasive procedure. All the non-invasive approaches on the other hand are only configured for stimulating large areas. With such approaches, it is not possible to stimulate deep brain areas without in addition stimulating areas nearer to the skull is.

SUMMARY OF TH EINVENTION

The invention provides for a medical apparatus, a computer program product and a method in the independent claims. Embodiments are given in the dependent claims.

Embodiments may provide a medical apparatus for generating a radio frequency electromagnetic field and focused ultrasound waves, which due to a limitation of the ratio of electric power density to acoustic power density maximizes the interaction of the electromagnetic field and the ultrasound waves. The maximization of the interactions allows to use lower electric power density and acoustic power density for which the risk of adverse effects may be minimized and/or excluded. This may be particularly advantageous for a headset, since the human brain is a particularly sensitive organ, which has to be protected against adverse effects caused e.g. by thermal heating or non-thermal negative pressure.

In one aspect, the invention provides for a medical apparatus. The medical apparatus comprises a headset with one or more coils for generating a radio frequency electromagnetic field and one or more ultrasound transmitters for generating focused ultrasound waves. Furthermore, the medical apparatus comprises a memory for storing machine executable instructions and a processor for controlling the medical apparatus.

Execution of the machine executable instructions causes the processor to control the one or more coils to generate the radio frequency electromagnetic field and the one or more ultrasound transmitters to generate the focused ultrasound waves. A ratio of electric power density of the electromagnetic field to acoustic power density of the ultrasound waves is limited to lie within a range from 0.5 to 2.

Embodiments may have the beneficial effect that a limitation of the ratio of electric power density and acoustic power density may be implemented which increases and/or maximizes the interaction of the electromagnetic field and the ultrasound waves. The resulting current densities are not required to large. For example, a current less than 1 mA may not induce firing of neuron directly, but may already modulate neural activities by changing the resting membrane potential, i.e. firing threshold.

The one or more coils generate a varying electromagnetic field which may induce an AC current. However, these radio frequency electrical current induced by the radio frequency electromagnetic field may not stimulate tissue, like brain tissue, directly, since its frequency is too high. But density variations resulting from the simultaneously generated ultrasound waves lead to conductivity variations which may locally rectify the AC current. The focused ultrasound waves may thus define a focused spot, the spatial extend of which limits a region in which tissue stimulation may arise. Using one or more coils for generating electromagnetic field that influences tissue by induction rather than applying a current directly with electrodes may reduce and/or avoid adverse effects, e.g. in the skin and skull bone. Operation frequencies may e.g. be in the range form 100 kHz to 500 kHz, in order to reduce specific absorption rate (SAR) issue.

Tissue, like brain tissue, is only responsive to electrical currents of sufficient low frequency. Thus, it is possible to apply rather large current to tissue at high frequencies. However, the limiting factor is the heat generated in the tissue. With a conductivity of the brain of about 0.2 S/m at 1 MHz, and an allowed maximum power density of 12 W/1, the possible current density is about 50 A/$m^2$ or about a total current of 10 A through the brain. This is a much higher current than usually applied for example in transcranial direct current stimulation (tDCS). However, at 1 MHZ, the resulting electrical effect may essentially be zero. To get an electrical effect, the induced AC current has to give rise to a near DC current.

This may be achieved by a rectification effect, wherein the resistivity for the current flowing in one direction is lower than flowing in the other direction. Such a rectification effect may be achieved by intense ultrasound waves. Ultrasound waves may change the density of the tissue. Known diagnostic ultrasound pressures are around $10^5$ Pa, i.e. about atmospheric pressure. For short duration, they may be increased tenfold. The compression modulus of water, which is comparable to the compression modulus of brain tissue, is about $2 \cdot 10^9$ Pa. Therefore, the change in density and conductivity is about $\pm 0.5 \cdot 10^{-4}$. This means, the rectifying effect is about $10^{-4}$ and a 10 A current at 1 MHz may result in a 1 mA near DC component.

Such a DC component may result in a DC stimulation for an efficient interaction of the electromagnetic filed and the ultrasound waves. If the ultrasound and electrical frequencies are slightly offset, also an AC stimulation may be achieved for a sufficient match between and control of the radio frequency electromagnetic field and the ultrasound waves. The spatial pattern of a near DC current flow is provided by alternating discs perpendicular to the travel direction of the ultrasound waves. The distance between such discs is about half a wave length, i.e. 0.7 mm at 1 MHz. The frequency of operation may be varied and therefore the distance between the disks. A lower frequency reduces the heating effect from ultrasound, while increasing the electric heating slightly. Since for stimulation, the focus region is larger than the usual resolution in diagnostic ultrasound, an application of the ultrasound through the skull may be less of a problem. Even with the distortions induced by the skull, a resolution of 1 cm may be easily achieved. The radio frequency AC current is generated by the one or more coils. Using magnetic induction has the advantage to produce less heating in the skull bone.

The power density to be deposit in a human head must not exceed 12 W/l and may be restricted to a maximum of about 4 W/l, i.e. about 4 kW/m$^3$ for brain tissue. The power density provided according to embodiments is given as a sum of acoustic power $P_a = k \cdot I$ generated by the one or more ultrasound transmitters and electric power $P_e = \rho \cdot \sigma^2$ generated by the one or more coils. Thus, the total power density is $P = \rho \cdot \sigma^2 + k \cdot I$, with $\rho$ the conductivity, $\sigma$ the current density, k a sound absorption constant and I the sound intensity. The sound intensity is given by $I = p^2/Z$ with p the sound pressure and Z the tissue impedance. The conductivity $\rho$ as well as the sound absorption constant k depend on the frequency of the electromagnetic field and ultrasound waves, respectively.

The low frequency current generation based on the radio frequency electromagnetic field and the ultrasound waves is proportional to $p \cdot \sigma$, so the lowest power dissipation may occur when both components, i.e. $P_a$ and $P_e$, each contribute about half of the power density deposited in the tissue, i.e. e.g. a maximum of 2 W/l for ultrasound heating and 2 W/l for electrical heating.

In order to operate in safe ultrasound conditions, the power dissipation may be restricted to a maximum of 2 W/l. The bone is the most critical structure for ultrasound absorption $k_{bone}$, wherein the $k_{bone}$ depends on the frequency. Depending on the frequency, the power flux at the skull may be limited to about 3 mW/cm$^2$ at 500 kHz and 9 mW/cm$^2$ at 100 kHz. After the skull, the ultrasound beam may be focused to roughly 100 times the power density, i.e. e.g. to 1.7 W/cm$^2$ for the 100 kHz case. The absorption in the brain is $k_{brain}$ depending on the frequency as well. The resulting maximum possible focal power may be limited to 18 mW/cm$^2$ at 500 kHz and 72 mW/cm$^2$ at 100 kHz. Thus, the full possibility of beam focusing cannot be utilized due to focal heating issues.

The power is linked to the pressure by $p = \sqrt{I \cdot Z}$. With a typical impedance of the brain matter of about $Z_{brain} = 1.6 \cdot 10^6$ kg/(s cm$^2$), the obtainable pres-

sures become for example 18 mW/cm$^2$ at 500 kHz a p = 0.17·10$^5$ Pa and 72 mW/cm$^2$ at 100 kHz a p = 0.34·10$^5$ Pa. The efficiency of converting radio frequency AC to (near) DC is about $\eta = p/G$ with G the compression module of tissue, i.e. about $G_{brain} = 2 \cdot 10^9$Pa under the assumption that the compression module for brain tissue is comparable to the compression module for water. Thus, the resulting conversion efficiency $\eta_{brain}$ may be given at 500 kHz by $\eta_{brain} = 0.85 \cdot 10^{-5}$ and at 100 kHz by $\eta_{brain} = 1.7 \cdot 10^{-5}$.

With a budget of $P_a = \rho \cdot \sigma^2 = 2$W/m$^3$ and the above conductivities, the following permissible current densities may be achieved at 500 kHz $\rho = 6.7 \ \Omega$ m à $\sigma$ = 17 A/cm$^2$ and at 100 kHz $\rho = 7.5 \ \Omega$ m a $\sigma$ = 16 A/cm$^2$. Multiplying the current densities with the rectifying efficiencies $\sigma_{DC} = \eta \ \sigma$ yields at 500 kHz a $\sigma_{DC} = 0.14$ mA/cm$^2$ and at 100 kHz a $\sigma_{DC} = 0.27$ mA/cm$^2$.

From the data presented, it may be seen that it is beneficial to use frequencies of less than 500 MHz. However, the frequencies should not become too low, since neurons become sensitive to current at low frequencies.

Using coils to generate a radio frequency electromagnetic field may be advantageous compared to known elements for applying a direct current using electrodes. The conductivity of the skull is only less than 10% of the brain conductivity. Therefore, it is not possible to apply the current by electrodes, but coils are needed to produced eddy currents in the brain.

According to embodiments, the ratio is limited to lie within a range from 0.67 to 1.5. According to embodiments, the ratio is limited to lie within a range from 0.83 to 1.2. According to embodiments, the ratio is 1. Embodiments may have the beneficial effect that the interaction is further optimized.

According to embodiments, the apparatus may comprise some form of input to time stimulation pulses with the task and some locking mechanism to avoid too high power dissipation.

According to embodiments, the frequency of the radio frequency electromagnetic field and the frequency of the focused ultrasound waves both lie within a range from 200 kHz to 300 kHz. According to embodiments, the frequencies of the radio frequency electromagnetic field and the frequency of the focused ultrasound waves both lie within a range from 225 kHz to 275 kHz.

Embodiments may have the beneficial effect that for frequency in the above identified range, the frequency depending interaction of electromagnetic field and ultrasound waves may be further optimized, while minimizing the risk of causing adverse effects.

According to embodiments, the frequency of the radio frequency electromagnetic field and the frequency of the focused ultrasound waves are equal. According to embodiments, the frequency of the radio frequency electromagnetic field and the frequency of the focused ultrasound waves are both 250 kHz.

According to embodiments, execution of the machine executable instructions causes the processor to repetitively activate and deactivate the one or more coils and the one or more ultrasound transmitters simultaneously, wherein within a predefined repetitive cycle an averaged share of time during which the one or more coils and the one or more ultrasound transmitters are active is limited to 25% percent of the total time duration of the repetitive cycle. According to embodiments, the averaged share of time during which the one or more coils and the one or more ultrasound transmitters are active is limited to 20%.

Embodiments may have the beneficial effect that due to the limitation of the share of time during which the coils and transmitters are active, a sufficient time for e.g. thermal dissipation may be ensured. Thus, adverse thermal effects may be effectively minimized.

For example, from trans-cranial direct current stimulation (tDCS), it may be derived that a current density of about 11 mA/m$^2$ is necessary to achieve an effect. In tDCS about 1 mA is applied to the skull, producing about 0.25 mA in the brain. At an effective area of 15·15 cm$^2$ this is equivalent to 11 mA/m$^2$. For even higher effects, the power requirement rises quadratic with CD current density as the sound pressure level cannot be raised due to occurrence of negative pressure and hence direct non-thermal tissue damage. Not only modulating brain activity but causing it needs 10 to 100 times the current density, i.e. 100 to 10000 times the power.

The above assumptions may be relaxed in that the limit for the maximum allowed power density for the total head is rather 12 W/l than 4 W/l. To even further raise the power density, time averaging may be performed as described above. Furthermore, a stronger pressure dependency of conductivity on pressure in tissue, like brain tissue, relative to water may further increase the efficiency of the apparatus.

According to embodiments, the averaged share of time during which the one or more coils and the one or more ultrasound transmitters are active within the repetitive cycle is provided by a continuous time span.

Embodiments may have the beneficial effect that a maximum continuous time span for thermal dissipation may be ensured.

According to embodiments, the total time duration of the repetitive cycle is 4 min to 6 min. According to embodiments, the total time duration of the repetitive cycle is 5 min. Embodiments may have the beneficial effect may have the beneficial effect that a resulting time spans for thermal dissipation may be optimized for brain tissue.

According to embodiments, the focused ultrasound waves are focused on a focus region with a maximum diameter in a range from 1°cm to 4°cm full-half-width or 1°cm to 3°cm full-half-width or 1°cm to 2°cm full-half-width. Embodiments may have the beneficial effect that the ultrasound waves are efficiently focused.

According to embodiments, the one or more coils and one or more ultrasound transmitters are located offset to one another at the headset. For example, the one or more coils and one or more ultrasound transmitters do not overlap one another. In case there is an overlap, the ultrasound transmitters are operated with reduced and/or no power in a region of maximum current density of the one or more coils. Embodiments may have the advantage in the region of maximum current density, which may be disadvantages in view of dissipation, ultrasound power is minimized.

According to embodiments, the one or more coils comprise litz wires. Embodiments may have the beneficial effect that a resistance in the coils may be efficiently minimized. Thus, allowing for higher currents in the coils.

Litz wire is a type of specialized multistrand wire used in electronics for carrying alternating current (AC) at radio frequencies. It is designed to reduce skin effect and proximity effect losses. Litz wire comprises a plurality of thin wire strands, individually insulated and twisted or woven together. According to embodiments, the litz wire may comprise several levels, i.e. groups of twisted/woven wires that are twisted/woven together. The resulting winding patters are designed such that the proportion of the overall length over which each strand is at the outside of the litz wire is equalized. Thus, the current may be distributed equally among the wire strands, reducing the resistance.

The resistance of a conductor depends on its cross-sectional area, i.e. a conductor with a larger area has a lower resistance for a given length. The skin effect refers to the fact that at high frequencies an alternating current does not penetrate deeply into a conductor due to eddy currents induced in the material, but rather tends to flow near the surface of the conductor. Since less current flows through the material near the center of the conductor, less of the cross-sectional area of the conductor is used and the resulting total AC resistance is greater than a DC resistance, i.e. the resistance in case of a direct current (DC) flowing through the same conductor without skin effect. The higher the frequency of the AC current, the smaller the depth to which the AC current penetrates. Thus, the AC resistance of wire increases with frequency.

A further effect influencing the resistance of a conductor and resulting in an additional increase in the resistance of the wire with frequency is the proximity effect. In applications, where multiple wires carrying the same current lie side-by-side, the magnetic field of the adjacent wires induces longitudinal eddy currents in each of the wires causing the current to be concentrated in narrow strips on the sides adjacent to the other adjacent wires. Like in case of the skin effect; the current is concentrated into smaller cross-sectional areas of the individual wires increasing the resistance.

A litz wire employs a stranded wire with individually insulated conductors forming a bundle. Each of the conductors used has a diameter less than a skin-depth, so individual strands do not suffer an appreciable skin effect loss. The depth to which AC current penetrates in a conductor is determined the skin depth, i.e. the depth at which the current density falls to 1/e of its surface value. The weaving or twisting pattern of the wires in the bundle is designed so that the individual strands are on the outside of the bundle for a distance where the EM field changes are smaller and the respective strand sees low resistance, and are inside for a distance where the EM field changes are the strongest and the resistance is higher. If each strand has a comparable impedance, current is distributed equally among every strand within the wire.

According to embodiments, the one or more coils comprise Aluminum wires. Embodiments may have the beneficial effect that coil dissipation for a given mass of the individual coils may be efficiently minimized. Aluminum provides for example a better electrical conductivity to weight ratio than other electrically conductive materials, like copper. Aluminum has approximately 59% of electrical conductivity of copper, while only having approximately 30% of density of copper. According to embodiments, the one or more coils comprise litz wires made of Aluminum.

According to embodiments, the one or more coils comprise saddle coils. A saddle coil refers to a coil with all its windings located in a single curved plain. Embodiments may have the beneficial effect that a saddle coil may allow to optimize the spatial distribution of the current density. Using at least one saddle-coil may ensure that at least one coil is incorporated in a shape to maximize the current density at the ultrasound focus region.

According to embodiments, the medical apparatus is powered by battery or a rechargeable battery pack. The head set may be provided with power by cable from the battery, which is worn in form of a separate battery pack.

According to embodiments, the arrangement of coils and/or transmitters comprised by the headset may be fixed. Such an embodiment may only be capable of addressing one specific region as focus position. The focus may be shifted only slightly e.g. by 2 to 3 cm. However, there may be two or more foci present simultaneously, e.g. for addressing both hemispheres simultaneously. So, in order to target different regions, different apparatuses may be required. According to alternative embodiments, the arrangement of coils and/or transmitters may be freely altered.

In another aspect, the invention comprises a computer program product. The computer program product comprises machine executable instructions for execution by a processor controlling a medical apparatus with a headset with one or more coils for generating a radio frequency electromagnetic field and one or more ultrasound transmitters for generating focused ultrasound waves.

Execution of the machine executable instructions causes the processor to control the one or more coils to generate the radio frequency electromagnetic field and the one or more ultrasound transmitters to generate the focused ultrasound waves, wherein a ratio of electric power density of the electromagnetic field to acoustic power density of the ultrasound waves is limited to lie within a range from 0.5 to 2.

According to embodiments the computer program product comprises machine executable instructions which are configured to enable the processor of the apparatus to control the same to implement any of the aforementioned functional features of the medical apparatus.

In another aspect, the invention comprises a method of operating a medical apparatus comprising a headset with one or more coils for generating a radio frequency electromagnetic field and one or more ultrasound transmitters for generating focused ultrasound waves, a memory storing machine executable instructions, and a processor for controlling the medical apparatus.

The method comprises executing the machine executable instructions by the processor. The execution causes the processor to control the one or more coils to generate the radio frequency electromagnetic field and the one or more ultrasound transmitters to generate the focused ultrasound waves. A ratio of electric power density of the electromagnetic field to acoustic power density of the ultrasound waves is limited to lie within a range from 0.5 to 2.

According to embodiments the method comprises executing machine executable instructions by the processor which are configured to enable the processor of the apparatus to control the same to implement any of the aforementioned functional features of the medical apparatus.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments, computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

BRIEF DESCRIPTION OF THE DRAWINGS

In the following preferred embodiments of the invention will be described, by way of example only, and with reference to the drawings in which:

Fig. 1 illustrates a first example of a medical apparatus; and
Fig. 2 illustrates a second example of a medical image.

DETAILED DESCRIPTION OF THE EMBODIMENTS

Like numbered elements in these figures are either equivalent elements or perform the same function. Elements which have been discussed previously will not necessarily be discussed in later figures if the function is equivalent.

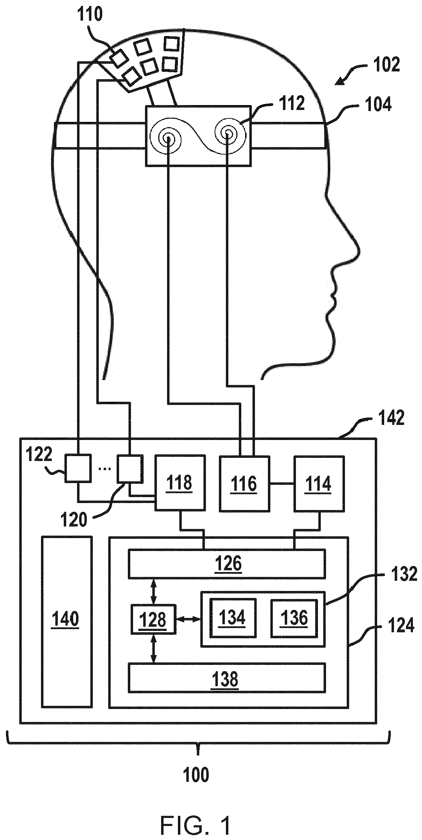

Fig. 1 illustrates an exemplary embodiment of a medical apparatus 100. The medical apparatus 100 comprises a headset 102 with one or more coils 112 for generating a radio frequency electromagnetic field and one or more ultrasound transmitters 110 for generating focused ultrasound waves. The headset 102 may comprise one or more fixation elements 104 for fixating the coils 112 and transmitters 110 to a human head. The fixation elements 104 may comprise one or more bands, straps etc. Furthermore or alternatively, the fixation elements 104 may comprise a helmet structure on and/or in which the coils 112 and transmitters 110 are arranged.

The ultrasound transmitters 110 shown in Fig. 1 are arrange in an exemplary 2D matrix structure. The one or more coils 112 may be provided in form of saddle coils. According to embodiments, the one or more coils 112 may be pairwise arranged on opposite sides of the headset 102, resulting in a pairwise arrangement on opposite sides of a head, when the headset 102 is located on the respective head. In Fig. 1, the one or more coils 112 and one or more ultrasound transmitters 110 are located offset to one another at the headset 102. Thus, the one or more coils 112 and one or more ultrasound transmitters 110 do not overlap one another. Alternatively, the one or more coils 112 and one or more ultrasound transmitters 110 may be located at the headset 102, such that they at least partially overlap each other. In case there is an overlap, the ultrasound transmitters 110 may be operated with reduced and/or no power in a region of maximum current density of the one or more coils 112. For the exemplary coil structure 112 shown in Fig. 1, this region of maximum current density is the region, where the winding direction of the coil 112 changes. According to embodiments, the one or more coils 112 may comprise litz wires and/or may comprise Aluminum wires.

The medical apparatus 100 comprises a control system 142 for controlling the coils 112 and transmitters 110 of the headset 102. The medical apparatus 110 may be configured as a mobile, wearable system. According to embodiments, the control system 142 or at least individual elements comprised by the control system142 shown in Fig. 1 are part of the headset 102. According to embodiments the control system 142 or at least individual elements by the control system 142 shown in Fig. 1 are provided as additional elements independent and/or separated of the headset 102. For example, the respective elements may be carried at a hip, back or chest using additional fixation elements, like e.g. a belt, harness or backpack. The respective elements may be operationally connected to the headset 102 e.g. by one or more leads, i.e. cables. According to embodiments, the operationally connect may additionally or alternatively comprise a wireless communication connection.

The control system 142 may comprise a computer system 124 and a power supply 140, like e.g. a rechargeable battery pack. The power supply 140 supplies the computer system 142 as well as the headset 102 and all the other energy consuming elements of the medical apparatus 100 with power.

The computer system 124 may comprise a hardware interface 126 to communicate with the other components of the apparatus 100, a processor 128, a computer memory 132 and a user interface 138. The user interface 138 may e.g. comprise a display. The user interface 138 may for example enable a user to switch the apparatus 100 on and off, receive status reports, set parameters and/or select predefined programs to be executed by the processor 128 in order to control the one or more coils 112 to generate the radio frequency electromagnetic field and the one or more ultrasound transmitters 110 to generate the focused ultrasound waves. The programs may e.g. define frequencies, power densities and/or sequences of activation and deactivation for the one or more coils 112 and ultrasound transmitters 110. The computer memory 132 may provide a control module 134 and a safety module 136. The control module 134 may comprise machine executable instructions to be executed by the processor 136. The machine executable instructions of the control module 134 may define parameters settings according to which the processor 136 controls the coils 112 and transmitters 110. The parameters may e.g. define an electric power density of the electromagnetic field to be generated by the coils 112 and an acoustic power density to be generated by the transmitters 110. Furthermore, the parameters may e.g. define one or more frequencies at which the coils 112 and transmitters 110 are to be operated and/or time spans and repetitive cycles of activation and deactivation of the coils 112 and transmitters 110. These parameters may e.g. be provided by one of the predefined programs selectable via the user interface 138. The safety module 136 may also comprise machine executable instructions to be executed by the processor 136. The machine executable instructions of the safety module 136 may define safety thresholds, wherein at least the coils 112 and transmitters 110 are automatically switched of, when one or more of the respective safety thresholds are reached. The safety thresholds may e.g. define a maximum power density which is allowed to be emitted by the apparatus during a predefined time span. The safety module 136 may e.g. be stored in an internal storage of the computer system 124. The control module 134 may also be stored in the internal storage of the computer system 124, it may be sent (via wire or wireless) to the computer system 124 from an additional external computer system or it may be provided on a non-transitory storage medium. The non-transitory storage medium may e.g. be provided in form of a removable media, like e.g. a memory card or USB flash drive. Furthermore, the computer system 124 may record the activities of the coils 112 and transmitters 110 according to the control executed by the processor 128 using the control module 134 in a log file.

The hardware interface 126 of computer system 124 is for example connected to an RF amplifier 115, a matching network 116 and the one or more coils 112. The matching network 116 may be used for optimizing the energy transfer via the coils, e.g. by matching the impedance of the one or more coils 112 to the impedance of the transmission line, i.e. the leads connecting the RF amplifier 115 and the coils 112. Furthermore, the hardware interface 126 of computer system 124 may be connected to an amplifier 118, one or more delay modules 120, 122, and the one or more ultrasound transmitters 110. An ultrasound beam comprising ultrasound waves generated by the one or more ultrasound transmitters 110 may be focused by properly delaying signals going to the individual transmitters 110 for transmission using the one or more delay modules 120, 122. For example, pulses exiting central transmitters may be delayed relative to pulses exiting peripheral transmitters, so that all ultrasound pulses arrive at a focus point and/or focus region simultaneously.

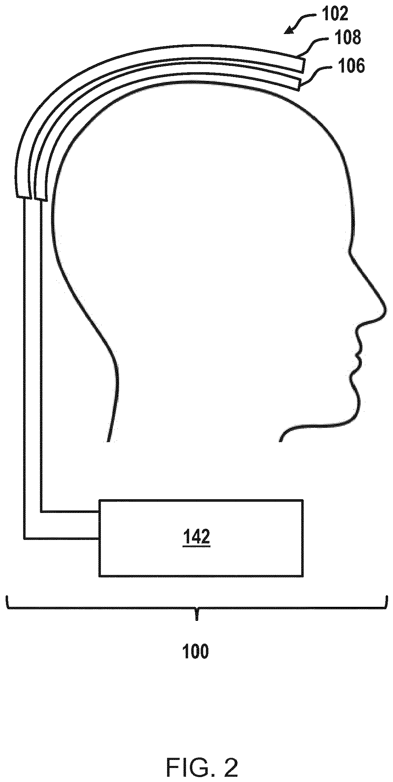

Fig. 2 illustrates a further exemplary embodiment of medical apparatus 100. The medical apparatus 110 shown in Fig. 2 may comprise the same control module 142 as the one shown un Fig. 1. The medical apparatus 100 of Fig.2 differs from the medical apparatus 100 shown in Fig.1 by the embodiment of the headset 102. The exemplary embodiment of the headset 102 in Fig. 2 has the form of a helmet. Fig. 2 shows a cross-sectional view of the helmet. The helmet comprises an inner shell 106 and an outer shell 108. The inner shell 106 may comprise the one or more ultrasound transmitters 110 in order to guaranty low-loss, direct ultrasound wave transmission. For example, the one or more ultrasound transmitters 110 may be arranged such that they are in direct contact with the head, when the helmet 102 is worn on the respective head. The outer shell may comprise the one or more coils 112, preventing them from direct contact with the head. The conductivity of the skull is only less than 10% of the brain conductivity. Therefore, it may be advantageous to not apply a current directly by electrodes contact with the head, but rather using coils 112 to produced eddy currents.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE NUMERALS

| | |
|---|---|
| 100 | medical apparatus |
| 102 | headset |
| 104 | fixation elements |
| 106 | inner shell |
| 108 | outer shell |
| 110 | ultrasound transmitter |
| 112 | coil |
| 114 | amplifier |
| 116 | matching network |
| 118 | amplifier |
| 120 | delay module |
| 122 | delay module |
| 124 | computer system |
| 126 | hardware interface |
| 128 | processor |
| 132 | computer memory |
| 134 | control module |
| 136 | safety module |
| 138 | user interface |
| 140 | battery |
| 142 | control system |

**Claims**

1. A medical apparatus (100) comprising:

   - a headset (102) with one or more coils (112) for generating a radio frequency electromagnetic field and one or more ultrasound transmitters (110) for generating focused ultrasound waves;
   - a memory (132) storing machine executable instructions (134);
   - a processor (128) for controlling the medical apparatus, wherein execution of the machine executable instructions causes the processor to control:

   the one or more coils to generate the radio frequency electromagnetic field and the one or more ultrasound transmitters to generate the focused ultrasound waves, wherein a ratio of electric power density of the electromagnetic field to acoustic power density of the ultrasound waves is limited to lie within a range from 0.5 to 2.

2. The medical apparatus of claim 1, wherein the ratio is limited to lie within a range from 0.67 to 1.5.

3. The medical apparatus of claim 2, wherein the ratio is limited to lie within the range of 0.83 to 1.2.

4. The medical apparatus of any one of the preceding claims, wherein the frequency of the radio frequency electromagnetic field and the frequency of the focused ultrasound waves both lie within a range from 200 kHz to 300 kHz.

5. The medical apparatus of claim 4, wherein the frequency of the radio frequency electromagnetic field and the frequency of the focused ultrasound waves both lie within a range from 225 kHz to 275 kHz.

6. The medical apparatus of any one of the preceding claims, wherein the frequency of the radio frequency electromagnetic field and the frequency of the focused ultrasound waves are equal.

7. The medical apparatus of any one of the preceding claims, wherein execution of the machine executable instructions causes the processor to repetitively activate and deactivate the one or more coils and the one or more ultrasound transmitters simultaneously, wherein within a predefined repetitive cycle an averaged share of time during which the one or more coils and the one or more ultrasound transmitters are active is limited to 25% percent of the total time duration of the repetitive cycle.

8. The medical apparatus of claim 7, wherein the averaged share of time during which the one or more coils and the one or more ultrasound transmitters are active is limited to 20%.

9. The medical apparatus of any one of claims 7 to 8, wherein the averaged share of time during which the one or more coils and the one or more ultrasound transmitters are active within the repetitive cycle is provided by a continuous time span.

10. The medical apparatus of any one of claims 7 to 9, wherein the total time duration of the repetitive cycle is 4 min to 6 min.

11. The medical apparatus of claim 10, wherein the total time duration of the repetitive cycle is 5 min.

12. The medical apparatus of any one of the preceding claims, wherein the headset comprises a helmet with an inner and an outer shell (106, 108), wherein the one or more ultrasound transmitters for generating focused ultrasound waves are located at the inner shell and the one or more coils for generating the radio frequency electromagnetic field are located at the outer shell.

13. A computer program product comprising machine executable instructions (134) for execution by a processor (128) controlling a medical apparatus (100), wherein the medical apparatus comprises a headset (102) with one or more coils (112) for generating a radio frequency electromagnetic field and one or more ultrasound transmitters (110) for generating focused ultrasound waves, wherein execution of the machine executable instructions causes the processor to control:
the one or more coils to generate the radio frequency electromagnetic field and the one or more ultrasound transmitters to generate the focused ultrasound waves, wherein a ratio of electric power density of the electromagnetic field to acoustic power density of the ultrasound waves is limited to lie within a range from 0.5 to 2.

14. A method of operating a medical apparatus (100), the medical apparatus comprising a headset (102) with one or more coils (112) for generating a radio frequency electromagnetic field and one or more ultrasound transmitters (110) for generating focused ultrasound waves, a memory (132) storing machine executable instructions (134), and a processor (128) for controlling the medical apparatus,
wherein the method comprises:
executing the machine executable instructions by the processor, causing the processor to control the one or more coils to generate the radio frequency electromagnetic field and the one or more ultrasound transmitters to generate the focused ultrasound waves, wherein a ratio of electric power density of the electromagnetic field to acoustic power density of the ultrasound waves is limited to lie within a range from 0.5 to 2.

FIG. 1

FIG. 2

Europäisches
Patentamt

European
Patent Office

Office européen
des brevets

## EUROPEAN SEARCH REPORT

Application Number

EP 18 20 7973

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 965 782 A1 (HIGHLAND INSTRUMENTS INC [US]) 13 January 2016 (2016-01-13) * paragraphs [0013] - [0014], [0026] - [0029], [0032]; figure 1 * ----- | 1-14 | INV. A61N1/40 A61N7/02 A61N1/04 A61N1/06 A61B90/50 A61N7/00 |
| A | US 5 476 438 A (EDRICH JOCHEN [DE] ET AL) 19 December 1995 (1995-12-19) * column 1, line 62 - column 2, line 31 * ----- | 1-14 | |
| A | US 2012/053391 A1 (MISHELEVICH DAVID J [US]) 1 March 2012 (2012-03-01) * paragraphs [0010], [0020] - [0021], [0032], [0035] - [0037]; figure 8 * ----- | 1-14 | |

TECHNICAL FIELDS
SEARCHED (IPC)

A61N
A61B

The present search report has been drawn up for all claims

| Place of search | Date of completion of the search | Examiner |
|---|---|---|
| Munich | 29 March 2019 | Monti, Adriano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 20 7973

This annex lists the patent family members relating to the patent documents cited in the above-mentioned European search report.
The members are as contained in the European Patent Office EDP file on
The European Patent Office is in no way liable for these particulars which are merely given for the purpose of information.

29-03-2019

| Patent document cited in search report | | Publication date | Patent family member(s) | | Publication date |
|---|---|---|---|---|---|
| EP 2965782 | A1 | 13-01-2016 | AU | 2007261108 A1 | 27-12-2007 |
| | | | CA | 2656023 A1 | 27-12-2007 |
| | | | CN | 101500644 A | 05-08-2009 |
| | | | EP | 2040790 A2 | 01-04-2009 |
| | | | EP | 2550992 A1 | 30-01-2013 |
| | | | EP | 2965782 A1 | 13-01-2016 |
| | | | JP | 5250549 B2 | 31-07-2013 |
| | | | JP | 2009540947 A | 26-11-2009 |
| | | | US | 2008046053 A1 | 21-02-2008 |
| | | | US | 2011264013 A1 | 27-10-2011 |
| | | | US | 2012004580 A1 | 05-01-2012 |
| | | | US | 2014378740 A1 | 25-12-2014 |
| | | | US | 2015105702 A1 | 16-04-2015 |
| | | | US | 2015119629 A1 | 30-04-2015 |
| | | | US | 2015151123 A1 | 04-06-2015 |
| | | | US | 2015343213 A1 | 03-12-2015 |
| | | | US | 2017296816 A1 | 19-10-2017 |
| | | | WO | 2007149811 A2 | 27-12-2007 |
| US 5476438 | A | 19-12-1995 | CN | 1093937 A | 26-10-1994 |
| | | | DE | 4408110 A1 | 15-09-1994 |
| | | | EP | 0617982 A1 | 05-10-1994 |
| | | | US | 5476438 A | 19-12-1995 |
| US 2012053391 | A1 | 01-03-2012 | NONE | | |

EPO FORM P0459

For more details about this annex : see Official Journal of the European Patent Office, No. 12/82